# FASCICULE DE BREVET EUROPEEN

(11) **EP 2 246 691 B1**
(45) Date de publication et mention de la délivrance du brevet: **13.07.2016**
(21) Numéro de dépôt: 10290220.2
(22) Date de dépôt: 27.04.2010
(51) Int. Cl.: G01N 15/14, G01N 33/49, G01N 33/86

(54) **Procédé et appareil de comptage des Thrombocytes**
Systeme und Verfahren zum Schätzen der Blutplättchenzahl
Method and device for platelets counting

(30) Priorité: 29.04.2009 FR 0902087
(43) Date de publication de la demande: 03.11.2010
(73) Titulaire: Commissariat à l'Énergie Atomique et aux Énergies Alternatives, 75015 Paris (FR)
(72) Inventeur: Faivre, Magalie, 38000 Grenoble (FR); Caillat, Patrice, 38000 Grenoble (FR); Cubizolles, Myriam-Laure, 38000 Grenoble (FR); Peponnet, Christine, 38170 Seyssinet (FR); Vauchier, Claude, 38120 St Egreve (FR)
(74) Mandataire: Gevers & Orès

(56) Documents cités:
- WO-A1-01/61324
- WO-A1-02/25280
- WO-A1-2007/068727
- WO-A1-2009/117682
- US-A- 6 133 995
- US-A1- 2002 160 523
- US-A1- 2006 024 756
- US-B1- 6 335 824
- US-B1- 6 391 568

## Description

L'invention porte sur un procédé de comptage des thrombocytes ou « plaquettes » dans un échantillon de sang par marquage fluorescent spécifique et traitement numérique d'images, ainsi que sur un appareil pour la mise en oeuvre d'un tel procédé.

Le procédé et l'appareil permettent en particulier le comptage des thrombocytes dans un échantillon de sang entier non lysé, peu ou pas dilué.

Les plaquettes ou thrombocytes sont des cellules sanguines de 2 à 5µm de diamètre sans noyau et de forme variable. Elles sont présentes dans le sang au nombre de 150.000 à 500.000/µL chez un sujet sain. Elles interviennent dans le processus d'hémostase primaire, et donc dans la coagulation. L'hémostase primaire permet d'arrêter le saignement dû à la lésion de capillaires, mais elle doit être renforcée par la coagulation si le calibre du vaisseau endommagé est plus important. Le rôle des thrombocytes est de s'agréger après activation et de sécréter diverses substances intervenant dans la coagulation comme la sérotonine, le calcium, l'adénosine diphosphate.contenus dans les granules denses du cytoplasme. Les plaquettes ont donc un rôle très important dans le phénomène de coagulation. Une diminution trop importante du nombre de plaquettes entraîne un risque hémorragique (à envisager avant une intervention chirurgicale par exemple). Une augmentation de leur nombre entraîne au contraire un risque de thrombose par formation d'agrégats plaquettaires. Les désordres plaquettaires peuvent être causés par une cirrhose, une insuffisance rénale (déficit de filtration du sang par les reins), une atteinte de la moelle osseuse, une infection virale ou encore une intoxication (Phénylbutazone, phénacétine, acide acétylsalicylique, etc.).

La méthode couramment utilisée pour compter les cellules sanguines, et en particulier les thrombocytes, est la cytométrie en flux, même si d'autres alternatives existent, comme la centrifugation.

Le principe de la cytométrie de flux est de compter les cellules en les faisant défiler une par une devant un système de mesure (optique ou électrique). Cela permet, à la fois de compter les cellules en détectant leur passage devant le système de mesure mais aussi de les caractériser (taille, conductivité, forme...). Pour permettre le passage des cellules une par une, de très forts facteurs de dilution sont appliqués à l'échantillon. Les deux types de cytométrie en flux sont la cytométrie optique (diffusion de lumière et/ou émission de fluorescence) et la cytométrie d'impédance. Des nombreux cytomètres en flux combinent les deux techniques pour permettre une mesure plus précise.

La centrifugation d'un échantillon sanguin dans un tube capillaire induit une séparation des différents types cellulaires selon leur densité. Les différents types cellulaires sont préalablement marqués spécifiquement. Les globules rouges se retrouvent au fond du tube, le plasma est au dessus. Entre les deux une zone appelée « couche leucocyto-plaquettaire » (« buffy-coat » en anglais) contient les plaquettes et les différents globules blancs. Un flotteur de densité adéquate est utilisé pour amplifier cette couche leucocyto-plaquettaire afin d'améliorer la précision. En mesurant les épaisseurs des différentes couches colorées, on en déduit le nombre de particules dans chaque couche donc le nombre de chaque type cellulaire.

Ces procédés nécessitent un équipement relativement lourd ; par conséquent, ils ne peuvent être mis en oeuvre que dans un laboratoire spécialisé. En outre, ils nécessitent de volumes relativement importants de sang (au moins de l'ordre de 100 µl).

Un procédé de comptage des thrombocytes très ancien, et désormais pratiquement désuet, comporte l'utilisation d'un hémocytomètre. L'hémocytomètre est un dispositif essentiellement constitué par une veine fluidique réalisée en verre, sur laquelle est gravée une grille de comptage. Un échantillon de sang lysé, dilué au moins 100 fois et dont les plaquettes sont marquées par un agent de contraste non spécifique du type colorant de Giemsa ou de Wright, est introduit dans l'hémocytomètre, puis observé en microscopie, ce qui permet le comptage manuel des cellules d'intérêt. Cette méthode a été pratiquement abandonnée en raison de sa précision insuffisante et du travail long et fastidieux qu'il demandait à un opérateur spécialisé pour identifier les plaquettes et effectuer le comptage manuel.

L'article de J.-S. Lin et al. «A PC-based imaging system for automated platelet identification », IEEE Transactions on Biomedical Engineering, Volume 39, N° 9, septembre 1992, pages 990 - 993 décrit un procédé d'indentification des plaquettes qui adhèrent à un substrat fonctionnalisé dans des conditions d'écoulement proches de celles rencontrées in vivo. Ce procédé, qui comporte un marquage fluorescent desdites plaquettes et l'utilisation d'un système informatique de traitement des images, ne permet pas de déterminer la concentration des plaquettes dans un échantillon sanguin car seules les cellules qui adhérent au substrat fonctionnalisé sont identifiées.

Les documents US 2002/160523 et WO 02/25280 décrivent des techniques de détections de thrombocytes basées sur la méthode connue sous l'acronyme "MLSC", signifiant "Microvolume Laser Scanning Cytometry", que l'on peut traduire comme "Cytométrie sur microvolume à balayage Laser". Cette méthode comporte l'introduction d'un échantillon de sang dilué dans un capillaire, son balayage par un faisceau laser focalisé et la détection d'un signal de fluorescence émis par des thrombocytes marqués. Cette technique par balayage qui utilise ainsi un éclairage non stationnaire est complexe et coûteuse à mettre en oeuvre.

Le document WO-A1-2007/068727 décrit une technique d'étude des processus de coagulation du sang pour étudier l'action de médicaments ayant un effet anti-thromboses, sans rechercher le comptage de thrombocytes.

Le document WO-A1-01/61324 décrit un microscope à fluorescence fonctionnant sans balayage, au moyen d'un éclairage stationnaire pour permettre une observation visuelle sans besoin de traitement informatique des images, et sans mentionner le comptage de thrombocytes.

Le document US-B1-6 335 824 décrit un microscope de fluorescence à balayage qui utilise une source de lumière stationnaire, contrairement à l'éclairage utilisé qui est rendu non stationnaire par l'intermédiaire d'un miroir oscillant.

Le document WO-A1-2009/117682 décrit un appareil de comptage de thrombocytes comportant une chambre d'analyse à paroi transparente dans laquelle est introduit un échantillon de sang mélangé à un colorant rendant les thrombocytes fluorescents sous un rayonnement déterminé, un décomposeur d'images de type capteur d'images « CCD » ou « CMOS » pour acquérir des images d'intensités de fluorescence émises par les thrombocytes et des moyens informatiques pour compter ces thrombocytes par référence à une intensité moyenne d'émission fluorescente.

L'invention vise à procurer un procédé simple et fiable de comptage des thrombocytes contenus dans un échantillon de sang, permettant de surmonter au moins certains des inconvénients de l'art antérieur.

Conformément à l'invention, ce but est atteint par un procédé de comptage des thrombocytes contenus dans un échantillon de sang, comportant :
- le mélange dudit échantillon avec :
   ▪ des marqueurs fluorescents susceptibles de se lier spécifiquement avec les thrombocytes ; et
   ▪ un agent inhibiteur de l'activation desdits thrombocytes ;
- l'introduction de l'échantillon (E) dans une chambre fluidique (CF) ayant au moins une face transparente, l'introduction étant mise en oeuvre :
   * soit après ledit mélange, lequel est alors introduit avec l'échantillon qu'il contient dans ladite chambre fluidique,
   * soit en même temps que ledit mélange, l'échantillon étant introduit dans ladite chambre fluidique qui contient au préalable à l'état sec lesdits marqueurs fluorescents et ledit agent inhibiteur de l'activation en vue de réaliser ledit mélange ;
- l'acquisition d'au moins une image numérique (IN) dudit mélange par microscopie à fluorescence sous éclairage stationnaire ; et
- le comptage des thrombocytes (T) présents dans ladite ou dans chaque image par des moyens informatiques de traitement des images (O).

Par « microscopie » on entend toute technique permettant l'observation d'objets microscopiques (c'est à dire non visibles à l'oeil nu), en particulier au moyen d'un système optique et d'un capteur d'images. Par « microscopie à fluorescence » on entend plus particulièrement toute technique de microscopie basée sur la détection d'un rayonnement de fluorescence émise par les objets à observer.

On parle d'« éclairage stationnaire » lorsque la zone éclairée de l'échantillon ne varie pas de manière perceptible au cours de l'acquisition d'image. Cela exclut notamment tout balayage de l'échantillon par un faisceau d'éclairage focalisé.

Selon des modes de réalisation particuliers du procédé de l'invention :
- Lesdits marqueurs fluorescents peuvent être des anticorps, eux-mêmes marqués par un fluorophore, susceptibles de se lier spécifiquement aux thrombocytes.
- Ledit agent inhibiteur de l'activation des thrombocytes peut être choisi entre : de la prostaglandine E1, du Dipyridamole, du Clopidogrel, de la Ticlopidine et de l'acide acétylsalicylique.
- Le mélange de l'échantillon de sang avec un anticoagulant peut également être prévu.
- L'échantillon de sang peut ne pas être dilué, sauf par ajout des réactifs nécessaires à la détection (anticoagulants, marqueurs, agent inhibiteur de l'activation). En variante, il peut être dilué d'un facteur inférieur ou égal à 50.
- Ladite chambre fluidique peut présenter une épaisseur comprise entre 15 µm et 60 µm.
- L'étape d'acquisition d'au moins une image numérique dudit échantillon peut être effectuée par microscopie à épifluorescence et/ou par un imageur comportant un capteur matriciel de type CCD ou CMOS.
- L'étape de comptage des thrombocytes peut comporter une étape de seuillage et binarisation des pixels de ladite ou de chaque image, suivie par une opération de comptage au sens propre.
- Le volume dudit échantillon de sang, avant ladite étape de mélange, peut être compris entre 1 et 100 µl.
- Ledit échantillon de sang peut être un échantillon de sang complet non lysé.
- Une étape consistant à déduire, du nombre de thrombocytes identifiées sur ladite ou chaque image numérique, un nombre de thrombocytes par unité de volume dans l'échantillon de sang peut également être prévue.
- De préférence, ladite chambre fluidique ne comporte pas de surfaces fonctionnalisées.
- De préférence, l'échantillon ne s'écoule pas à l'intérieur de ladite chambre fluidique.

Un autre objet de l'invention est un appareil de comptage des thrombocytes comportant :
- une chambre fluidique dans laquelle peut être introduit un échantillon de sang, comportant une paroi au moins partiellement transparente et contenant : un agent inhibiteur de l'activation desdits thrombocytes ; et des marqueurs fluorescents susceptibles de se lier spécifiquement avec lesdits thrombocytes ;
- une source de lumière pour réaliser un éclairage stationnaire dudit échantillon de manière à exciter lesdits marqueurs fluorescents ;
- un système optique (MEF) de microscopie à fluorescence pour acquérir au moins une image numérique de fluorescence (IN) dudit échantillon ; et
- des moyens informatiques de traitement des images adaptés pour compter de manière automatique les thrombocytes présents dans ladite ou chaque image de fluorescence.

D'autres caractéristiques, détails et avantages de l'invention ressortiront à la lecture de la description faite en référence aux dessins annexés donnés à titre d'exemple et qui représentent, respectivement :
- la figure 1, le principe de la méthode ; et
- la figure 2, un graphique illustrant ses performances.

Le principe du procédé repose sur l'acquisition et l'analyse informatique d'images, acquises au microscope, d'un échantillon de sang dont les thrombocytes ont été inactivés et marqués de manière spécifique.

Ci-après sera exposé, à titre d'exemple, un protocole de mise en oeuvre d'un tel procédé.

Un échantillon de sang entier est prélevé sur un anticoagulant tel que l'EDTA (acide éthylène-diamine-tétraacétique) déposé à sec à une concentration de 1,8 mg/ml, ou du citrate de sodium liquide à la concentration de 1 volume pour 9 volumes de sang. Puis, un volume de 5µl de cet échantillon est mélangé avec 45µl de tampon salin (PBS - « phosphate buffer saline »), le diluant ainsi 10 fois. La dilution n'est pas essentielle à la mise en oeuvre, mais elle facilite le comptage des thrombocytes par analyse d'images en augmentant leur séparation moyenne et en évitant ainsi des effets de superposition pouvant conduire à une sous-estimation du nombre effectif de cellules.

Un volume de 0,5 µl de prostaglandine E1 est ajouté pour inactiver les thrombocytes et empêcher ainsi leur agrégation.

Ensuite, 10 µl d'anticorps marqué (anti-CD61 FITC à 0,5 mg/ml) sont ajoutés à la solution pour marquer de manière spécifique les thrombocytes. L'anti-CD61 est un anticorps susceptible de se lier aux thrombocytes, et la FITC (Fluorescéine Iso Thio Cyanate) est un fluorophore d'utilisation courante.

Le mélange est homogénéisé par aspirations et largages successifs à l'aide d'une micropipette µP ; puis il est laissé à incuber à température ambiante, dans l'obscurité, pendant 15 minutes.

Il est intéressant de noter que l'utilisation d'anticoagulant n'est pas indispensable si on limite à environ 3 - 5 minutes le temps d'incubation, ce qui semble être suffisant pour réaliser le marquage. Au contraire, l'adjonction de prostaglandine E1 ou d'un autre inhibiteur de l'activation des thrombocytes s'est avérée essentielle. En effet, en son absence les thrombocytes s'activent et, même en présence d'un anticoagulant, forment des agrégats, ce qui empêche leur comptage.

Après incubation, l'échantillon E obtenu par mélange du sang avec le diluant et les différents réactifs (anticoagulant, anti-activant, anticorps marqué) est introduit dans une chambre fluidique CF ayant au moins une face transparente, qui peut être constituée par deux lames de verre de microscope, L1 et L2, réunies par l'intermédiaire de deux bandes adhésives BA de manière à délimiter une veine fluidique ayant une épaisseur de l'ordre de 30 µm. Le remplissage peut s'effectuer, très simplement, par capillarité.

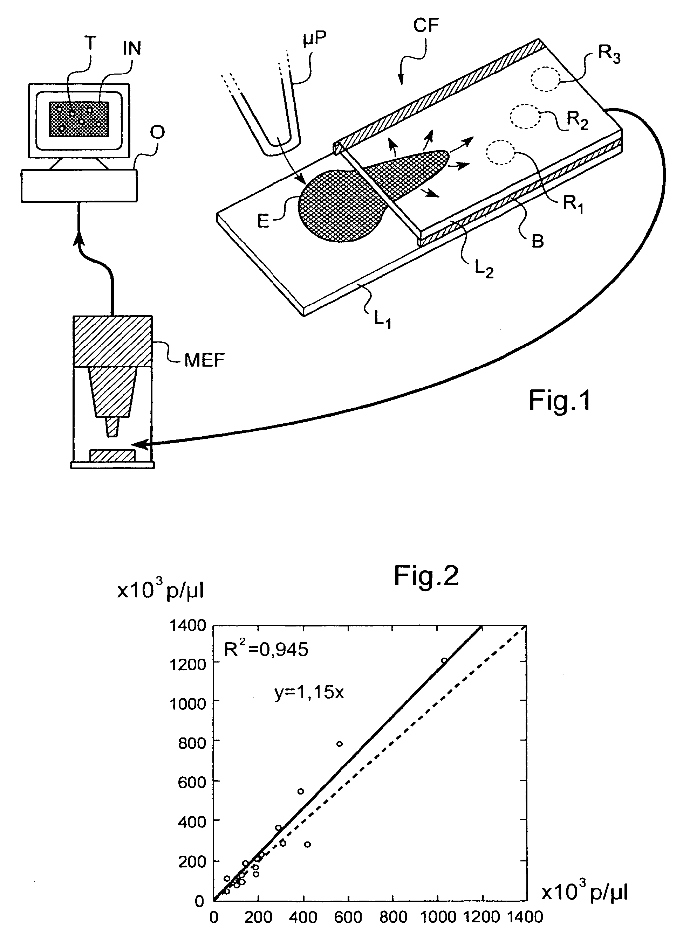

Ensuite la chambre fluidique - ou plutôt une région de cette chambre - est observée dans un microscope à épifluorescence MEF de type conventionnel, comportant une source de lumière d'excitation des fluorophores et un système optique d'acquisition d'images de fluorescence, relié à un ordinateur O. Il est possible d'effectuer plusieurs observations à plusieurs endroits différents de la chambre (par exemple, les trois régions R1, R2 et R3 sur la figure 1) pour compenser d'éventuelles inhomogénéités résiduelles.

Lorsque l'échantillon est maintenu, sans écoulement, dans la chambre, une ou plusieurs images numériques IN sont acquises, dans lesquelles les thrombocytes marqués apparaissent comme des points lumineux T, isolés grâce à l'agent anti-activant qui empêche leur agrégation, sur un fond plus sombre. Ensuite, la ou les images sont traitées par un ordinateur de la manière suivante :
- leur contraste est rehaussé, et leur luminosité est réglée par l'utilisateur ;
- un seuil de binarisation est défini, de manière à convertir les images d'un format à niveaux de gris à un format binaire noir et blanc ; et
- le nombre d'objets (points blancs, ou noirs si on travaille en négatif) est compté directement, de manière automatique.

En faisant l'hypothèse que chaque objet sur l'image représente un et un seul thrombocyte, on détermine ainsi un nombre de cellules pour chaque fenêtre d'observation. La moyenne des valeurs ainsi déterminées est enfin convertie en nombre de cellules par unité de volume grâce à l'estimation du volume de la chambre fluidique correspondant à une fenêtre d'observation du microscope.

L'ensemble de la mesure prend environ 20 minutes, du prélèvement de l'échantillon de sang à l'obtention du comptage plaquettaire. Ce temps peut encore être réduit en abrégeant la période d'incubation.

Le procédé de l'invention a été comparé avec un comptage de thrombocytes obtenu par cytométrie de flux à l'aide d'un analyseur « Automate Coulter LH 780 Hematology Analyzer » combinant une mesure optique et une mesure électrique.

Les mesures de référence en cytométrie de flux ont été effectuées sur des échantillons de 200 µl de sang total prélevé sur EDTA, dilués d'un facteur 6250. Pour le procédé de l'invention, le protocole décrit ci-dessus a été appliqué. On remarquera que l'invention permet d'utiliser des quantités de sang bien plus faibles que la cytométrie de flux.

La figure 2 permet de comparer les valeurs de numération plaquettaire obtenus par la méthode de référence (axe des abscisses, x) et par la méthode de l'invention (axe des ordonnées, y) pour 17 échantillons de sang total issus de patients différents.

La corrélation est linéaire : y=1,15x, avec un bon coefficient de régression linéaire (R²=0,945) ; la répétabilité des mesures est satisfaisante (Coefficient de Variation ou CV, représentant l'écart type de la mesure divisée par la moyenne, exprimé en pourcentage inférieur ou égal à 10% pour une concentration de plaquettes comprise entre 50.000 µl⁻¹ et 1000000 µl⁻¹)). Pour comparaison, la variation de la méthode de référence, donnée par le CV, est comprise entre 3,3 % et 6,6, selon les gammes de concentration plaquettaire (3.3 % entre 280000 µl⁻¹ et 320000 µl⁻¹ - 6.6% entre 90000 µl⁻¹ et 110000 µl⁻¹). Ces résultats pourraient être améliorés en utilisant des chambres fluidiques d'épaisseurs plus homogènes.

L'hématocrite des échantillons analysés varie entre 25% et 46% ; il a été vérifié que cette variation du taux d'hématocrite n'a pas d'influence significative sur les résultats obtenus.

Le procédé de l'invention a été décrit en référence à un mode de réalisation particulier ; cependant, des nombreuses modifications peuvent être envisagées. En particulier :
Tout anticoagulant connu peut être utilisé en remplacement ou en complément de l'EDTA, par exemple le citrate de sodium ou l'héparine.

Comme discuté plus haut, la dilution de l'échantillon n'est pas essentielle, mais elle permet d'utiliser un traitement d'images particulièrement simple en réduisant la probabilité que deux ou plusieurs plaquettes se superposent sur l'image. Si on souhaite travailler sans dilution, il est nécessaire de prévoir un traitement d'images plus complexe, permettant de mesurer la surface des objets fluorescents enregistrés pour déterminer s'ils correspondent à une ou plusieurs plaquettes.

Le taux de dilution ne devrait pas non plus être trop élevé afin de ne pas dégrader la statistique du comptage, étant donné que le procédé de l'invention prévoit l'observation de très faibles volumes de sang. Typiquement, des taux de dilution compris entre 0 (pas de dilution) et 50 et de préférence de l'ordre de 10 s'avèrent adaptés à la mise en oeuvre de l'invention.

Le diluant peut être du PBS (Phosphate Buffer Saline en anglais ou tampon salin phosphaté), d'une solution de thyorode mélangé à de l'acide 4-(2-hydroxyethyl)piperazine-1-ethanesulfonique, du trishydroxyméthyl-aminométhane, ou tout autre tampon salin qui maintient l'osmolarité de l'échantillon et le pH à des valeurs physiologiques.

L'anti-activant peut être de la Prostaglandine E1, du Dipyridamole, du Clopidogrel, de la Ticlopidine, de l'acide acétylsalicylique (ou aspirine). Tout autre anti-agrégeant plaquettaire peut être utilisé, ayant par exemple comme mode d'action :
- l'augmentation des taux des nucléotides cycliques ;
- l'inhibition du métabolisme de l'acide arachidonique ;
- l'inhibition des récepteurs membranaires ;
- l'inhibition des processus d'activation (inhibition des canaux calciques, inhibition de la calmoduline, inhibition du cytosquelette, ...) ;
- l'inhibition de la formation et/ou de l'action de la thrombine ;

L'anticorps de marquage pourra être choisi de manière à maximiser l'affinité avec les cellules d'intérêt. Pour cela, le clone le plus afin doit être choisi.

Comme discuté plus haut, le temps d'incubation doit avantageusement être le plus court possible, tout en assurant le marquage de la quasi-totalité des plaquettes. Si ce temps d'incubation est suffisamment court, l'utilisation d'un anticoagulant peut s'avérer superflue.

Le fluorophore couplé à l'anticorps pourra être varié, par exemple l'iso thio cyanate de fluoresceine (ou FITC), la protéine de peridinine chlorophylle (ou PerCP), la R-phycoérythrine (ou R-PE), etc.

L'épaisseur de la veine fluidique peut être comprise de préférence entre 15 µm et 60 µm. Une épaisseur plus fine pourrait engendrer des problèmes de remplissage car la hauteur de la chambre serait du même ordre de grandeur que la taille des plus grandes cellules sanguines. Une épaisseur trop importante introduirait un risque de chevauchement des plaquettes sur l'image et, de ce fait, demanderait d'utiliser une optique trop résolutive pour visualiser les plaquettes dans l'épaisseur de la veine.
Dans le mode de réalisation décrit en détail, la chambre fluidique été constituée par deux lames de verre superposées et réunies par des bandes adhésives. Ces lames de verre sont de préférence non fonctionnalisées. En effet, le but de l'invention n'est pas la détermination du nombre de plaquettes ayant adhéré sur une surface délimitée, mais plutôt une énumération de l'ensemble des plaquettes comprises dans l'échantillon analysé.

Bien entendu, tout autre dispositif microfluidique adapté peut être utilisé. En particulier, la chambre fluidique peut être intégrée à une puce microfluidique. Ce qui est nécessaire est qu'au moins certaines zones de la face supérieure soient transparentes aux longueurs d'onde d'excitation et d'émission des fluorophores pour permettre l'observation des plaquettes marquées. D'autres matériaux biocompatibles que le verre peuvent être utilisés, tels que le polycarbonate.

D'autres techniques d'imagerie à fluorescence que la microscopie à épifluorescence peuvent être utilisées.

Une régulation de la température de l'échantillon peut également être prévue.

Lorsque le taux de dilution est faible, par exemple inférieur à 5, ou en cas d'absence de dilution, (taux de dilution égal à 0), l'hypothèse selon laquelle, sur l'image de fluorescence, chaque point lumineux correspond à un et un seul thrombocyte, peut aboutir une sous-estimation de la détermination du nombre de thrombocytes. En effet, les thrombocytes étant plus concentrés, les signaux de fluorescence peuvent se superposer. Cette sous-estimation peut-être atténuée en adoptant un traitement d'image plus raffiné, tenant compte de la forme et/ou de la surface de chaque point détecté.

Un dispositif selon l'invention est composé d'une source de lumière d'excitation. Selon un mode préféré, cette source de lumière est susceptible d'éclairer de manière stationnaire (et donc sans balayage) une. partie significative de la surface de l'échantillon. Par significative, on entend au moins 10% et de préférence au moins 20% ou 30%, voire la totalité de la surface. Cette source d'excitation n'est pas focalisée. Il peut notamment s'agir d'une lampe à incandescence ou d'une diode à électroluminescence. On utilise de préférence un filtre en longueur d'onde placé entre la source de lumière et l'échantillon, afin que la lumière atteignant l'échantillon corresponde à la longueur d'onde d'excitation du label fluorescent utilisé.

La source peut-être placée par exemple à une distance de l'échantillon comprise entre 1 mm et 10 cm. Elle est de préférence statique, c'est-à-dire placée de façon fixe face à l'échantillon. Elle émet un rayonnement autour d'un axe fixe. En effet, comme expliqué plus haut, le procédé de l'invention ne nécessite pas de balayage du rayonnement produit par la source. Cela permet de réduire le cout et la complexité de sa mise en oeuvre.

Le détecteur comprend de préférence un capteur d'images, c'est à dire un capteur matriciel pixellisé, dont le champ couvre une partie significative de l'échantillon, voire la totalité de l'échantillon. Ce capteur permet, en une durée très courte (quelques centaines de ms à quelques s), d'obtenir une image de fluorescence du champ observé. Il peut notamment s'agir d'un capteur comprenant un objectif relié à une matrice CCD ou CMOS. Ce capteur peut-être placé du même côté de la source par rapport à l'échantillon (on parle alors de mesure en réflexion), ou du côté opposé (on parle alors de mesures en transmission).

Ainsi, un dispositif selon l'invention comprend de préférence d'une source de lumière fixe et non collimatée, un capteur d'images matriciel, et une chambre microfluidique. Il s'agit d'un dispositif peu cher, transportable, et de conception simple, ne nécessitant aucun mouvement de la source et du détecteur par rapport à l'échantillon observé.

## Revendications

1. Procédé de comptage des thrombocytes contenus dans un échantillon de sang, comportant :
- le mélange dudit échantillon avec :
o des marqueurs fluorescents susceptibles de se lier spécifiquement avec les thrombocytes ; et
o un agent inhibiteur de l'activation desdits thrombocytes ;
- l'introduction de l'échantillon (E) dans une chambre fluidique (CF) ayant au moins une face transparente, l'introduction étant mise en oeuvre :
* soit après ledit mélange, lequel est alors introduit avec l'échantillon qu'il contient dans ladite chambre fluidique,
* soit en même temps que ledit mélange, l'échantillon étant introduit dans ladite chambre fluidique qui contient au préalable à l'état sec lesdits marqueurs fluorescents et ledit agent inhibiteur de l'activation en vue de réaliser ledit mélange ;
- l'acquisition d'au moins une image numérique (IN) dudit mélange par microscopie à fluorescence sous éclairage stationnaire ; et
- le comptage des thrombocytes (T) présents dans ladite ou dans chaque image par des moyens informatiques de traitement des images (O).

2. Procédé de comptage des thrombocytes selon la revendication 1, dans lequel lesdits marqueurs fluorescents sont des anticorps, eux-mêmes marqués par un fluorophore, susceptibles de se lier aux thrombocytes.

3. Procédé de comptage des thrombocytes selon l'une des revendications précédentes, dans lequel ledit agent inhibiteur de l'activation des thrombocytes est choisi entre: de la prostaglandine E1, du Dipyridamole, du Clopidogrel, de la Ticlopidine et de l'acide acétylsalicylique.

4. Procédé de comptage des thrombocytes selon l'une des revendications précédentes, comportant également le mélange de l'échantillon de sang avec un anticoagulant.

5. Procédé de comptage des thrombocytes selon l'une des revendications précédentes, dans lequel l'échantillon de sang n'est pas dilué.

6. Procédé de comptage des thrombocytes selon l'une des revendications 1 à 4 comportant également la dilution de l'échantillon de sang d'un facteur inférieur ou égal à 50.

7. Procédé de comptage des thrombocytes selon l'une des revendications précédentes, dans lequel ladite chambre fluidique présente une épaisseur comprise entre 15 µm et 60 µm.

8. Procédé de comptage des thrombocytes selon l'une des revendications précédentes, dans lequel l'étape d'acquisition d'au moins une image numérique dudit échantillon est effectuée par microscopie à épifluorescence.

9. Procédé de comptage des thrombocytes selon l'une des revendications précédentes, dans lequel l'étape de comptage des thrombocytes comporte une étape de seuillage et binarisation des pixels de ladite ou de chaque image, suivie par une opération de comptage au sens propre.

10. Procédé de comptage des thrombocytes selon l'une des revendications précédentes, dans lequel le volume dudit échantillon de sang, avant ladite étape de mélange, est compris entre 1 et 100 µl.

11. Procédé de comptage des thrombocytes selon l'une des revendications précédentes, dans lequel ledit échantillon de sang est un échantillon de sang entier non lysé.

12. Procédé de comptage des thrombocytes selon l'une des revendications précédentes, comportant également une étape consistant à déduire, du nombre de thrombocytes identifiés sur ladite ou chaque image numérique, un nombre de thrombocytes par unité de volume dans l'échantillon de sang.

13. Procédé de comptage des thrombocytes selon l'une des revendications précédentes, dans lequel ladite chambre fluidique ne comporte pas de surfaces fonctionnalisées.

14. Procédé de comptage des thrombocytes selon l'une des revendications précédentes, dans lequel l'échantillon ne s'écoule pas à l'intérieur de ladite chambre fluidique.

15. Appareil de comptage des thrombocytes comportant :
- une chambre fluidique (CF) dans laquelle peut être introduit un échantillon de sang, comportant une paroi au moins partiellement transparente et contenant :
* un agent inhibiteur de l'activation desdits thrombocytes, et
* des marqueurs fluorescents susceptibles de se lier spécifiquement avec lesdits thrombocytes ;
- une source de lumière pour réaliser un éclairage stationnaire dudit échantillon de manière à exciter lesdits marqueurs fluorescents ;
- un système optique d'acquisition d'images de microscopie à fluorescence pour acquérir au moins une image numérique de fluorescence (IN) dudit échantillon ; et
- des moyens informatiques de traitement des images (O) adaptés pour compter de manière automatique les thrombocytes (T) présents dans ladite ou chaque image de fluorescence.

## Patentansprüche

1. Verfahren zum Zählen der Thrombozyten, die in einer Blutprobe enthalten sind, aufweisend:
- das Mischen der Probe mit:
∘ fluoreszierenden Markern, die imstande sind, sich speziell mit den Thrombozyten zu verbinden, und
∘ einem Inhibitor der Aktivierung der Thrombozyten,
- das Einbringen der Probe (E) in eine Fluidkammer (CF) mit mindestens einer transparenten Seite, wobei das Einbringen durchgeführt wird:
* entweder nach dem Mischen, wobei die Mischung dann mit der Probe, die sie enthält, in die Fluidkammer eingebracht wird,
* oder gleichzeitig mit dem Mischen, wobei die Probe in die Fluidkammer eingebracht wird, die bereits die fluoreszierenden Marker und den Inhibitor der Aktivierung in trockenem Zustand enthält, um das Mischen durchzuführen,
- das Erfassen mindestens eines digitalen Bilds (IN) der Probe durch Fluoreszenzmikroskopie unter stationärer Beleuchtung und
- das Auszählen der in dem oder in jedem Bild vorhandenen Thrombozyten (T) mit Hilfe von EDV-Bildverarbeitungsmitteln (0).

2. Verfahren zum Zählen der Thrombozyten nach Anspruch 1, wobei die fluoreszierenden Marker selbst durch ein Fluorophor markierte Antikörper sind, die imstande sind, sich an die Thrombozyten zu binden.

3. Verfahren zum Zählen der Thrombozyten nach einem der vorangehenden Ansprüche, wobei der Inhibitor der Aktivierung der Thrombozyten aus dem Prostaglandin E1, Dipyridamol, Clopidogrel, dem Ticlopidin und der Acetylsalicylsäure ausgewählt ist.

4. Verfahren zum Zählen der Thrombozyten nach einem der vorangehenden Ansprüche, umfassend ebenfalls das Mischen der Blutprobe mit einem Antikoagulans.

5. Verfahren zum Zählen der Thrombozyten nach einem der vorangehenden Ansprüche, wobei die Blutprobe nicht verdünnt ist.

6. Verfahren zum Zählen der Thrombozyten nach einem der Ansprüche 1 bis 4, umfassend ebenfalls das Verdünnen der Blutprobe mit einen Faktor unter oder von gleich 50.

7. Verfahren zum Zählen der Thrombozyten nach einem der vorangehenden Ansprüche, wobei die Fluidkammer eine Dicke zwischen 15 µm und 60 µm inklusive aufweist.

8. Verfahren zum Zählen der Thrombozyten nach einem der vorangehenden Ansprüche, wobei der Schritt des Erfassens mindestens eines digitalen Bilds von der Probe durch Epifluoreszenzmikroskopie durchgeführt wird.

9. Verfahren zum Zählen der Thrombozyten nach einem der vorangehenden Ansprüche, wobei der Schritt des Zählens der Thrombozyten einen Schritt der Segmentierung und Binarisierung der Pixel des oder jedes Bilds, gefolgt von einem Zählvorgang im eigentlichen Sinne, aufweist.

10. Verfahren zum Zählen der Thrombozyten nach einem der vorangehenden Ansprüche, wobei das Volumen der Blutprobe vor dem Schritt des Mischens zwischen 1 und 100 µl inklusive beträgt.

11. Verfahren zum Zählen der Thrombozyten nach einem der vorangehenden Ansprüche, wobei die Blutprobe eine nicht lysierte Probe ganzen Bluts ist.

12. Verfahren zum Zählen der Thrombozyten nach einem der vorangehenden Ansprüche, aufweisend ebenfalls einen Schritt, der darin besteht, aus der Anzahl von auf dem oder jedem digitalen Bild identifizierter Thrombozyten eine Anzahl von Thrombozyten je Volumeneinheit in der Blutprobe abzuleiten.

13. Verfahren zum Zählen der Thrombozyten nach einem der vorangehenden Ansprüche, wobei die Fluidkammer keine funktionalisierten Oberflächen aufweist.

14. Verfahren zum Zählen der Thrombozyten nach einem der vorangehenden Ansprüche, wobei die Probe nicht in das Innere der Fluidkammer fließt.

15. Vorrichtung zum Zählen der Thrombozyten, umfassend:
- eine Fluidkammer (CF), in die eine Blutprobe einbringbar ist, aufweisend eine mindestens teilweise transparente Wand und enthaltend:
* einen Inhibitor der Aktivierung der Thrombozyten und
* fluoreszierende Marker, die imstande sind, sich speziell an die Thrombozyten zu binden,
- eine Lichtquelle, um eine stationäre Beleuchtung der Probe derart durchzuführen, dass die fluoreszierenden Marker angeregt werden,
- ein optisches System der Fluoreszenzmikroskopie-Bilderfassung zum Erfassen mindestens eines digitalen Fluoreszenzbilds (IN) der Probe und
- EDV-Bildverarbeitungsmittel (0), die ausgebildet sind, um die in dem oder in jedem Fluoreszenzbild vorhandenen Thrombozyten (T) automatisch zu zählen.

## Claims

1. A method of counting thrombocytes contained in a sample of blood, the method comprising:
• mixing said sample with:
• fluorescent markers suitable for bonding specifically with the thrombocytes; and
• an agent for inhibiting activation of said thrombocytes;
• introducing the sample (E) into a fluidic chamber (CF) having at least one transparent face, the introduction being carried out:
* either after said mix, which is introduced with the sample it contains in said fluidic chamber,
* or simultaneously with said mix, the sample being introduced in said fluidic chamber containing beforehand in a dry state said fluorescent markers and said agent for inhibiting activation in order to produce said mix;
• acquiring at least one digital image (IN) of said sample by fluorescence microscopy under stationary illumination; and
• counting the thrombocytes (T) present in said or each image by image processing computer means (0).

2. A method of counting thrombocytes according to claim 1, wherein said fluorescent markers are antibodies, themselves marked with a fluorophore, and suitable for bonding with thrombocytes.

3. A method of counting thrombocytes according to any preceding claim, wherein said thrombocyte activation inhibitor agent is selected from: prostaglandin E1, dipyridamole, clopidogrel, ticlopidine, and acetyl salicylic acid.

4. A method of counting thrombocytes according to any preceding claim, also including mixing the sample of blood with an anticoagulant.

5. A method of counting thrombocytes according to any preceding claim, wherein the sample of blood is not diluted.

6. A method of counting thrombocytes according to any one of claims 1 to 4, also including diluting the sample of blood by a factor less than or equal to 50.

7. A method of counting thrombocytes according to any preceding claim, wherein said fluidic chamber presents thickness lying in the range 15 µm to 60 µm.

8. A method of counting thrombocytes according to any preceding claim, wherein the step of acquiring at least one digital image of said sample is performed by epifluorescence microscopy.

9. A method of counting thrombocytes according to any preceding claim, wherein the step of counting thrombocytes includes a step of thresholding and binarizing the pixels of the or each image, followed by an operation of counting proper.

10. A method of counting thrombocytes according to any preceding claim, wherein the volume of said sample of blood, prior to said mixing step, lies in the range 1 µL to 100 µL.

11. A method of counting thrombocytes according to any preceding claim, wherein said sample of blood is a sample of non-lyzed whole blood.

12. A method of counting thrombocytes according to any preceding claim, also including a step that consists in deducing a number of thrombocytes per unit volume in the sample of blood from the number of thrombocytes identified in said or each digital image.

13. A method of counting thrombocytes according to any preceding claim, wherein said fluidic chamber does not comprise any functionalized surface.

14. A method of counting thrombocytes according to any preceding claim, wherein the sample does not flow inside said fluidic chamber.

15. Apparatus for counting thrombocytes, the apparatus comprising:
• a fluidic chamber (CF) into which a sample of blood can be introduced, the chamber having a wall that is at least partially transparent and containing:
* an agent for inhibiting activation of said thrombocytes, and
* fluorescent markers suitable for bonding specifically with said thrombocytes;
• a source of light for performing stationary illumination of said sample in such a way as to excite said fluorescent markers;
• a fluorescence microscopy optical system (MEF) for acquiring at least one digital image (IN) of the fluorescence of said sample; and
• computer means (0) for image processing adapted to count the thrombocytes (T) present in said or each fluorescence image in automatic manner.
